# EUROPEAN PATENT APPLICATION

(11) **EP 4 585 140 A1**
(43) Date of publication of application: **16.07.2025**
(21) Application number: 23862926.5
(22) Date of filing: 22.08.2023
(51) Int. Cl.: A61B 3/028, A61B 3/08

(54) **SUBJECTIVE OPTOMETRY SYSTEM AND SUBJECTIVE OPTOMETRY PROGRAM**

(30) Priority: 09.09.2022 JP 2022143454
(71) Applicant: NIDEK CO., LTD., Gamagori-shi, Aichi 443-0038 (JP)
(72) Inventor: HORINO, Taeko, Gamagori-shi Aichi 443-0038 (JP); SHIMAZAKI, Arisa, Gamagori-shi Aichi 443-0038 (JP)
(74) Representative: Hoefer & Partner Patentanwälte mbB
(86) International application number: PCT/JP2023/030102
(87) International publication number: WO 2024/053387

(57) **Abstract**

A subjective optometry system is for subjectively measuring optical characteristics of a subject eye. The subjective optometry system includes a target presentation unit which presents an examination visual target to the subject eye, a correction unit which changes an optical characteristic of a target light flux emitted from the target presentation unit, a self-optometry program setting unit which sets either a first self-optometry program that automatically proceeds with examination items for the subject eye based on a response input by an examinee, or a second self-optometry program that automatically proceeds with examination items for the subject eye based on a response input by the examinee and has a different examination condition from the first self-optometry program, and a control unit which controls, based on the first self-optometry program or the second self-optometry program set by the self-optometry program setting unit, at least one of the target presentation unit and the correction unit to advance an optometry according to a procedure of the first self-optometry program or the second self-optometry program.

## Description

### TECHNICAL FIELD

The present disclosure relates to a subjective optometry system and a subjective optometry program for subjectively measuring optical characteristics of a subject eye.

### BACKGROUND ART

In a subjective optometry system, a subjective optometry device is used for subjectively measuring optical characteristics of a subject eye. For example, the subjective optometry device can measure the optical characteristics of the subject eye by placing an optical member in front of the subject eye and presenting a visual target to the subject eye via the optical member. For example, an examinee uses the subjective optometry device to perform self-optometry in which the examinee advances a subjective examination by himself or herself (see Patent Literature 1).

### CITATION LIST

### PATENT LITERATURE

Patent Literature 1: JP2022-089255A

### SUMMARY OF INVENTION

In the self-performed optometry using such a subjective optometry device, an examination is advanced according to a self-optometry program that takes into account examination conditions necessary to smoothly perform the self-optometry. However, an examination status of the subject eye (for example, experience of an examiner, optical characteristics of the subject eye, and the like) and an operation status (for example, a congestion status, an examination schedule, and the like) of a facility or a store in which the device is installed vary widely, and a preset self-optometry program may not suitable in some cases.

In view of the above problems, an object of the present disclosure is to provide a subjective optometry system and a subjective optometry program capable of easily executing a self-optometry program suitable for a situation in which self-optometry is performed on an subject eye.

A subjective optometry system according to a first aspect of the present disclosure is for subjectively measuring optical characteristics of a subject eye. The subjective optometry system includes: a target presentation unit configured to present an examination visual target to the subject eye; a correction unit configured to change an optical characteristic of a target light flux emitted from the target presentation unit; a self-optometry program setting unit configured to set either a first self-optometry program that automatically proceeds with a plurality of examination items for the subject eye based on a response input by an examinee, or a second self-optometry program that automatically proceeds with a plurality of examination items for the subject eye based on a response input by the examinee, the second self-optometry program having a different examination condition from the first self-optometry program; and a control unit configured to control, based on the first self-optometry program or the second self-optometry program set by the self-optometry program setting unit, at least one of the target presentation unit and the correction unit to advance an optometry according to a procedure of the first self-optometry program or the second self-optometry program.

A subjective optometry program according to a second aspect of the present disclosure is used in a subjective optometry system for subjectively measuring optical characteristics of a subject eye. The subjective optometry system includes a target presentation unit configured to present an examination visual target to the subject eye, and a correction unit configured to change an optical characteristic of a target light flux emitted from the target presentation unit. The subjective optometry program includes: a self-optometry program setting step of setting either a first self-optometry program that automatically proceeds with a plurality of examination items for the subject eye based on a response input by an examinee, or a second self-optometry program that automatically proceeds with a plurality of examination items for the subject eye based on a response input by the examinee, the second self-optometry program having a different examination condition from the first self-optometry program; and a control step of controlling, based on the first self-optometry program or the second self-optometry program set in the self-optometry program setting step, at least one of the target presentation unit and the correction unit to advance an optometry according to a procedure of the first self-optometry program or the second self-optometry program.

### BRIEF DESCRIPTION OF DRAWINGS

[FIG. 1A] FIG. 1A is an external view of an eye refractive power measurement unit supported at a standby position.
[FIG. 1B] FIG. 1B is an external view of the eye refractive power measurement unit supported at a measurement position.
[FIG. 2A] FIG. 2A is a schematic diagram illustrating an optical arrangement of a light projection optical system during a distance vision examination.
[FIG. 2B] FIG. 2B is a schematic diagram illustrating an optical arrangement of the light projection optical system during a near vision examination.
[FIG. 3] FIG. 3 is a diagram illustrating a polarizing optical member.
[FIG. 4] FIG. 4 is a schematic diagram of the eye refractive power measurement unit.
[FIG. 5] FIG. 5 is a schematic diagram of a control system of a subjective optometry device.
[FIG. 6] FIG. 6 is an example of a self-optometry assistance screen when a first self-optometry program is set.
[FIG. 7] FIG. 7 is an example of a self-optometry assistance screen when a second self-optometry program is set.
[FIG. 8A] FIG. 8A is a diagram illustrating how a polarized red-green visual target appears to both eyes.
[FIG. 8B] FIG. 8B is a diagram illustrating how the polarized red-green visual target appears to a left eye.
[FIG. 8C] FIG. 8C is a diagram illustrating how the polarized red-green visual target appears to a right eye.
[FIG. 9] FIG. 9 is an example of a self-optometry assistance screen in a second example.
[FIG. 10] FIG. 10 is an example of a schedule setting screen in the second example.
[FIG. 11] FIG. 11 is an example of a self-optometry program selection screen in the second example.
[FIG. 12] FIG. 12 is an example of a self-optometry assistance screen for editing a self-optometry program.
[FIG. 13] FIG. 13 is an example of the self-optometry program selection screen.
[FIG. 14] FIG. 14 is an example of a self-optometry program editing screen.
[FIG. 15] FIG. 15 is a diagram in which a plurality of subjective optometry devices are connected to a network via a terminal device.

### DESCRIPTION OF EMBODIMENTS

### <Overview>

An outline of a subjective optometry system according to the present embodiment will be described. Note that items grouped in < > below can be used independently or in conjunction with each other.

The subjective optometry system according to the present embodiment is a system for subjectively measuring optical characteristics of a subject eye. For example, the optical characteristics of a subject eye may be eye refractive power (as an example, at least one of spherical power, cylindrical power, astigmatic axis angle, and the like), a binocular vision function (as an example, at least one of a prism amount, a stereoscopic vision function, and the like), contrast sensitivity, and the like.

The subjective optometry system according to the present embodiment may include a target presentation unit (for example, 31). The target presentation unit presents an examination visual target to a subject eye. For example, the target presentation unit presents the examination visual target to the subject eye by emitting a target light flux toward the subject eye.

For example, the target light flux from the target presentation unit may be directly guided toward the subject eye. Further, for example, the target light flux from the target presentation unit may be guided toward the subject eye via a light projection optical system (for example, a light projection optical system 30). For example, the light projection optical system may include at least one optical member for passing the target light flux emitted from the target presentation unit. As an example, at least one of a lens, a mirror, and the like may be provided.

The subjective optometry system according to the present embodiment may include a correction unit. The correction unit changes an optical characteristic of the target light flux emitted from the target presentation unit. For example, the correction unit may be disposed in an optical path of the light projection optical system to change the optical characteristic of the target light flux.

The correction unit may have any configuration as long as the optical characteristic of the target light flux can be changed.

For example, the correction unit may include an optical element. For example, the optical element may be at least one of a spherical lens, a cylindrical lens, a variable focus lens, a cross cylinder lens, a rotary prism, a wavefront modulating element, and the like. Of course, the optical element may be different from these. In this case, the optical characteristic of the target light flux are changed by controlling the optical element.

Further, for example, the correction unit may have a configuration for optically changing a presenting position (presenting distance) of the visual target with respect to the subject eye. As an example, the correction unit may have a configuration that moves the target presentation unit in an optical axis direction, or may have a configuration that moves an optical element (for example, a spherical lens) in the optical path in the optical axis direction. In this case, the optical characteristic of the target light flux are changed by controlling a drive unit for controlling at least one of the target presentation unit and the optical element.

Further, for example, the correction unit may be an eye refractive power measurement unit (for example, an eye refractive power measurement unit 40) that switches and positions an optical element (for example, an optical element 52) via an examination window (for example, an examination window 43) in front of the subject eye. For example, the eye refractive power measurement unit may include a lens disk (for example, a lens disk 50) in which a plurality of optical elements are arranged on the same circumference. In this case, the optical characteristic of the target light flux are changed by controlling a drive unit (for example, a drive unit 51, a drive unit 53, and the like) for controlling the lens disk.

The subjective optometry system according to the present embodiment may include a self-optometry program setting unit (for example, a control unit 60). The self-optometry program setting unit sets either a first self-optometry program or a second self-optometry program which has a different examination condition from the first self-optometry program. The first self-optometry program automatically proceeds with a plurality of examination items for the subject eye based on a response input by an examinee, and the second self-optometry program automatically proceeds with a plurality of examination items for the subject eye based on the response input by the examinee. For example, the examination item may be an item indicating the type of examination to be performed in a subjective examination of the subject eye. For example, the examination item may be an item provided for each type of optical characteristics of the subject eye, and may be an item for obtaining an examination result for a predetermined optical characteristic of the subject eye.

The subjective optometry system may include a selection unit for selecting and setting the self-optometry program. For example, the selection unit may be configured to select either the first self-optometry program or the second self-optometry program. As an example, at least one of a check button, a radio button, a pull-down menu, a selection switch provided in a device housing, and the like may be used. Accordingly, the examiner can set any self-optometry program.

For example, in the present embodiment, a difference in the examination condition between the first self-optometry program and the second self-optometry program may be at least one of a difference in a condition related to an examination item, a difference in a condition related to an examination visual target, and the like. Of course, a combination of differences in the condition related to the examination item and the condition related to the examination visual target may be used.

For example, in the first self-optometry program and the second self-optometry program, the difference in the condition related to the examination item may be at least one of an examination item to be performed in each self-optometry program, an order in which a plurality of examination items are performed, a parameter for determining the examination result for a predetermined examination item (as an example, the highest visual acuity value of the subject eye), and the like.

For example, the first self-optometry program and the second self-optometry program may have different examination items. That is, the first self-optometry program and the second self-optometry program may include different combinations of the plurality of examination items. For example, a plurality of examination items included in the first self-optometry program and a plurality of examination items included in the second self-optometry program may be totally different from each other. Further, for example, at least one examination item among the plurality of examination items included in the first self-optometry program and the plurality of examination items included in the second self-optometry program may be the same. As an example, in this case, in addition to the plurality of examination items included in the first self-optometry program, a plurality of examination items in the second self-optometry program may be set to include an examination item different from these. As a result, it is possible to easily set the self-optometry program corresponding to a type of glasses desired by the examinee, an examination status of the subject eye (for example, experience of an examiner, optical characteristics of the subject eye, and the like), an operation status (for example, busy times and schedule) of a facility or a store in which the subjective optometry system is installed, or the like, and to proceed with each examination item.

Further, for example, in the first self-optometry program and the second self-optometry program, the difference in the condition related to the examination visual target may be at least one of the number of examination visual targets used in the examination item included in each self-optometry program, the type of the examination visual target (for example, Landolt ring visual target and Tumbling E visual target), a display format of the visual acuity value of the examination visual target (for example, decimal display and logarithmic display), and the like. The difference in the examination condition between the first self-optometry program and the second self-optometry program may include a difference in the content of the voice guide that accompanies differences in conditions related to such examination item and examination visual target.

For example, the first self-optometry program and the second self-optometry program may have different examination visual targets used in the examination item. That is, the examination visual target used in the examination item common to the first self-optometry program and the second self-optometry program may be different. For example, in the examination for the subject eye, the type of the examination visual target or the display format of the visual acuity value may be changed to suit the country or region in which the subjective optometry system is installed, or the number or the type of the examination visual targets may be changed according to the examination status of the subject eye. As in the present embodiment, since a plurality of self-optometry programs having different examination visual targets are provided in advance, the self-optometry program can be switched as necessary, and the examination can be smoothly performed.

In the present embodiment, the plurality of examination items included in each self-optometry program may include an examination item for examining the visual acuity of the subject eye. As an example, the examination item may be a naked eye visual acuity examination for examining the naked eye visual acuity of the subject eye, a corrected visual acuity examination for examining the corrected visual acuity of the subject eye, and the like.

In the present embodiment, the plurality of examination items included in each self-optometry program may include an examination item for examining the refractive power of the subject eye. As an example, the examination item may include at least one of a spherical examination for examining the spherical refractive power of the subject eye, a cylindrical examination for examining the cylindrical refractive power of the subject eye, an astigmatic axis examination for examining the astigmatic axis angle of the subject eye, and the like. Of course, all of the spherical examination, the cylindrical examination, and the astigmatic axis examination may be included.

Further, in the present embodiment, the plurality of examination items included in each self-optometry program may include an examination item for examining the binocular vision function of the subject eye. As an example, the examination item may include at least one of a binocular accommodation balance examination for examining balance of accommodation of the subject eye, a heterophoria examination for examining heterophoria of the subject eye, a strabismus examination for examining the strabismus of the subject eye, a convergence examination for examining the convergence of the subject eye, a fusion examination for examining the fusion of the subject eye, a stereoscopic vision examination for examining the stereoscopic vision of the subject eye, and the like. Of course, all of the binocular accommodation balance examination, the heterophoria examination, the strabismus examination, the convergence examination, the fusion examination, and the stereoscopic vision examination may be included.

The plurality of examination items included in each self-optometry program may be a combination of at least one of the examination item for examining the visual acuity of the subject eye, the examination item for examining the refractive power of the subject eye, the examination item for examining the binocular vision function of the subject eye, and the like. Of course, all of these examination items may be included.

In the present embodiment, the first self-optometry program may include an examination item for examining the refractive power of the subject eye as the plurality of examination items, and the second self-optometry program may include an examination item for examining the binocular vision function of the subject eye in addition to the examination item for examining the refractive power of the subject eye as the plurality of examination items. For example, the examination item for examining the refractive power of the subject eye is a general examination item. For example, when glasses are made for a subject eye, an examination item for examining the refractive power is executed. Therefore, by setting the first self-optometry program, it is possible to efficiently cope with many examinees. In addition, for example, the examination item for examining the binocular vision function of the subject eye is an examination item performed when it is desired to grasp the optical characteristics of the subject eye. Therefore, by setting the second self-optometry program, it is possible to make glasses that are more optimal for the subject eye by using the examination results of the refractive power and the visual function of the subject eye.

The subjective optometry system according to the present embodiment may include a control unit (for example, the control unit 60). The control unit controls at least one of the target presentation unit and the correction unit based on the first self-optometry program or the second self-optometry program set by the self-optometry program setting unit, and proceeds with the optometry according to a procedure of the first self-optometry program or the second self-optometry program. For example, the control unit may control the target presentation unit based on the first self-optometry program or the second self-optometry program to change at least one of the type of the examination visual target displayed on the target presentation unit, the visual acuity value of the examination visual target, and the like. Further, for example, the control unit may control the correction unit based on the first self-optometry program or the second self-optometry program to change at least one of the correction power for correcting the subject eye, the arrangement of the optical element, and the like. Accordingly, it is possible to smoothly advance the examination according to the program selected according to the type of glasses desired by the examinee, the examination status of the subject eye, the operation status of the facility or store in which the subjective optometry system is installed, and the like.

The subjective optometry system according to the present embodiment may include a setting change unit (for example, the control unit 60). For example, the setting change unit changes at least one of the examination condition of the first self-optometry program and the examination condition of the second self-optometry program. That is, for example, by using the setting change unit, it is possible to customize at least one of the examination condition of the first self-optometry program and the examination condition of the second self-optometry program. For example, the setting change unit may be configured to change, among an initial examination condition in the first self-optometry program (first examination condition) and an initial examination condition in the second self-optometry program (second examination condition), only the first examination condition, or only the second examination condition. Of course, for example, the setting change unit may be configured to change both the first examination condition and the second examination condition. For example, depending on the examinee, the examination conditions of the first self-optometry program and the second self-optometry program provided in advance in the subjective optometry system may not be suitable. Therefore, by changing the examination conditions using the setting change unit, the examination can be smoothly performed under desired examination conditions.

The subjective optometry system according to the present embodiment may include an optometry schedule setting unit (for example, the control unit 60). The optometry schedule setting unit sets an optometry schedule in which the first self-optometry program and the second self-optometry program are associated with a predetermined period. For example, the predetermined period may be a regular period such as a predetermined date or a predetermined time (for example, morning and afternoon, a time zone designated by the examiner, or the like). Further, for example, the predetermined period may be a regular period based on calendar information such as a day of the week, a week, and a month. Accordingly, it is possible to set the optometry schedule according to an operation status of a facility or a store in which the subjective optometry system is installed. For example, in such a case, the self-optometry program setting unit sets to switch between the first self-optometry program and the second self-optometry program, based on the optometry schedule set by the optometry schedule setting unit. Since each self-optometry program is automatically switched every predetermined period, the examination can be smoothly performed.

The subjective optometry system according to the present embodiment may include an operation unit (for example, an examiner controller 10). The operation unit inputs an operation signal for setting at least one of the first self-optometry program and the second self-optometry program. In this case, the optometry schedule setting unit sets, based on the operation signal from the operation unit, an optometry schedule in which the first self-optometry program and the second self-optometry program are associated with a predetermined period. Accordingly, for example, an administrator of a facility or a store in which the subjective optometry system is installed or an examiner using the subjective optometry system can set an optimal optometry schedule for each facility or store.

Of course, for example, the operation signal for setting the optometry schedule may be input by receiving an operation signal from a management medium (for example, a computer) that manages the subjective optometry system. Further, for example, the operation signal for setting the optometry schedule may be input by using an external storage unit (as an example, an SD card, a USB memory, a server, a cloud, or the like). In this case, the control unit included in the subjective optometry system may input an operation signal according to the setting by reading the setting of the optometry schedule stored in the external storage unit. For example, when such a management medium or an external storage unit is used, the contents of the first self-optometry program and the second self-optometry program, the content of the optometry schedule, and the like can be easily unified in a facility or a store in which the subjective optometry system is introduced.

The present disclosure is not limited to the devices described in the present embodiment. For example, terminal control software (program) for performing the functions of the above-described embodiment may be supplied to a system or a device via a network or various storage media, and a control device (for example, a CPU) of the system or the device may read and execute the program.

### <First Example>

A first example of the subjective optometry system according to the present embodiment will be described. In the present example, as an example of the subjective optometry system, a subjective optometry device for subjectively measuring optical characteristics of a subject eye, which integrally includes a display that displays an examination visual target to the subject eye and an eye refractive power measurement unit that changes an optical characteristic of a target light flux emitted from the display, will be described. Of course, for example, the display may be provided as a housing separate from the subjective optometry device.

### <Appearance of Device>

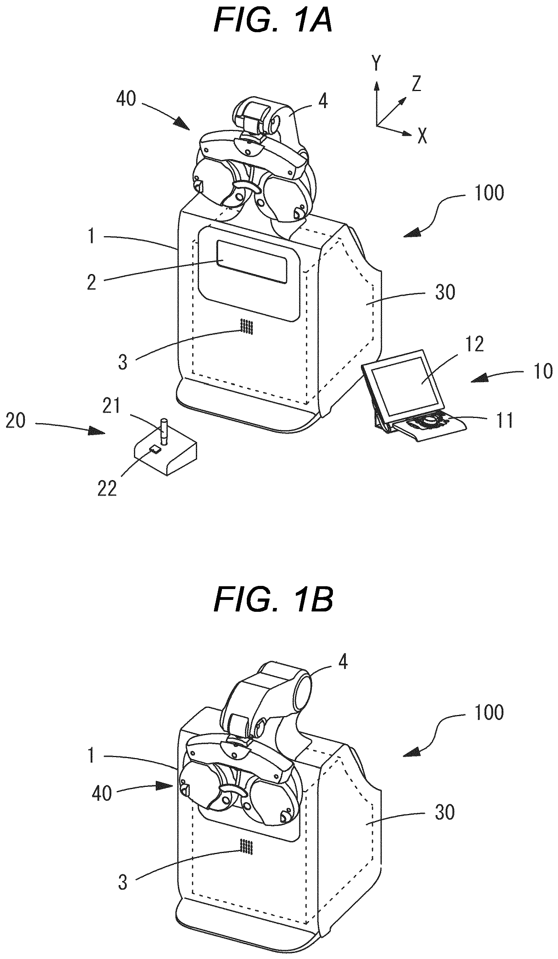

FIGS. 1A and 1B are external views of a subjective optometry device 100. FIG. 1A shows a state in which an eye refractive power measurement unit 40 is supported at a standby position. FIG. 1B shows a state in which the eye refractive power measurement unit 40 is supported at a measurement position. For example, the subjective optometry device 100 includes a housing 1, a presentation window 2, a speaker 3, a holding unit 4, an examiner controller 10, an examinee controller 20, the eye refractive power measurement unit 40, and the like.

The housing 1 includes a light projection optical system 30 therein. The presentation window 2 transmits a target light flux by the light projection optical system 30. The target light flux is projected onto a subject eye E through the presentation window 2. When the eye refractive power measurement unit 40 is disposed between the subject eye E and the presentation window 2 (see FIG. 1B), the target light flux is projected on the subject eye E through the presentation window 2 and the examination window 43 to be described later. Accordingly, the examination visual target is presented to the subject eye E. The speaker 3 outputs a voice guide or the like.

The holding unit 4 holds the eye refractive power measurement unit 40. For example, the holding unit 4 moves an arm by driving a drive unit (such as a motor) (not shown) to move the eye refractive power measurement unit 40 coupled to the arm. This allows the eye refractive power measurement unit 40 to be switched between the standby position and the measurement position.

The examiner controller 10 is used by an examiner to operate the subjective optometry device 100. The examiner controller 10 includes a switch unit 11, a monitor 12, and the like. The switch unit 11 inputs a signal for performing various settings (for example, movement of the eye refractive power measurement unit 40). The monitor 12 displays a self-optometry assistance screen to be described later. For example, various types of information (for example, an examination result of the subject eye E) may be displayed on the self-optometry assistance screen. The monitor 12 may function as a touch panel that also serves as the switch unit 11. A signal from the examiner controller 10 is output to the control unit 60 by wired communication or wireless communication.

The examinee controller 20 is used to input a response of the examinee. The examinee controller 20 includes a response lever 21, a response button 22, and the like. The response lever 21 is used when the examinee inputs a direction of an examination visual target. For example, signals in four directions of up, down, left, and right can be input by a tilting operation. The response button 22 is used when the examinee does not select a direction of the examination visual target. A signal from the examinee controller 20 is output to the control unit 60 by wired communication or wireless communication.

### <Light Projection Optical System>

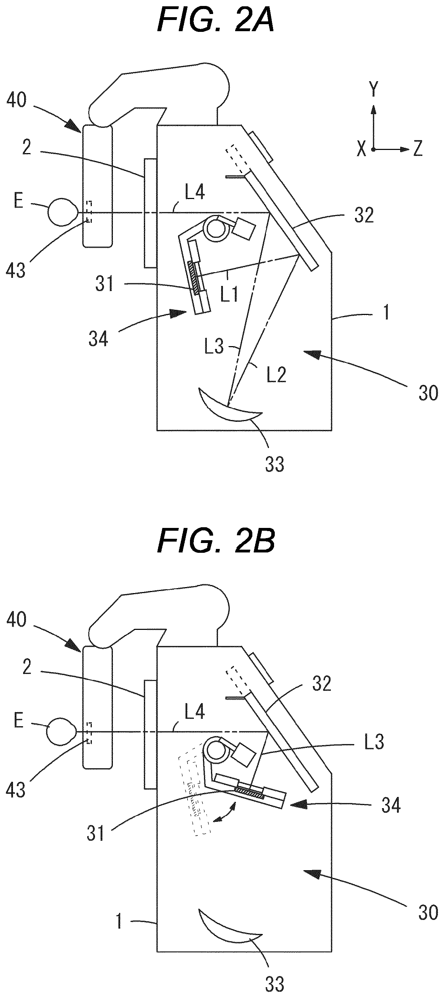

FIGS. 2A and 2B are schematic diagrams of the light projection optical system 30. FIG. 2A shows an optical arrangement at the time of a distance vision examination. FIG. 2B shows an optical arrangement at the time of a near vision examination. The light projection optical system 30 projects a target light flux toward the subject eye E. For example, the light projection optical system 30 includes a display 31, a flat mirror 32, a concave mirror 33, and a distance-near switching unit 34.

The display 31 displays a visual target (for example, a fixation visual target, an examination visual target, or the like). The target light flux emitted from the display 31 forms an image on the fundus of the subject eye E, so that a visual target is presented to the subject eye E. For example, the display 31 may be a liquid crystal display (LCD), an organic electro luminescence (EL), a plasma display, or the like.

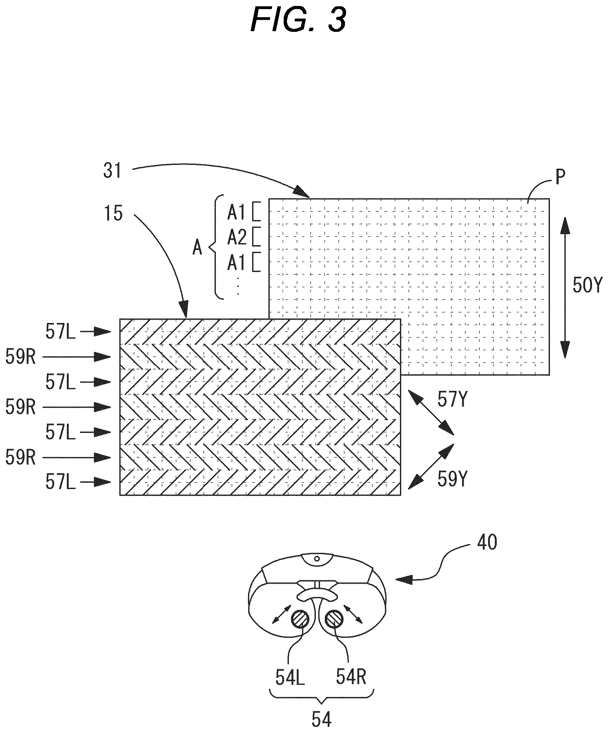

The display 31 may include a polarizing optical member. For example, the polarizing optical member may be disposed on a front surface of the display 31, or the polarizing optical member may be incorporated and integrally disposed in the display 31. Thus, the display 31 can emit linearly polarized light having a polarization axis in a predetermined direction (vertical, horizontal, 45-degree diagonal, or the like). In the present example, a target light flux having a polarization axis (a polarization axis indicated by an arrow 50Y in FIG. 3) in the vertical direction is emitted from the display 31.

FIG. 3 is a diagram illustrating the polarizing optical member 15. For example, the polarizing optical member 15 includes left-eye optical regions 57L and right-eye optical regions 59R that are alternately arranged in a line shape or a lattice shape on the front surface of the display 31. Among pixels P of the display 31, the left-eye optical region 57L is arranged in association with a first pixel region A1 of at least two adjacent pixels, and the right-eye optical region 59R is arranged in association with a second pixel region A2 of at least two adjacent pixels. In this example, the left-eye optical regions 57L and the right-eye optical regions 59R are alternately arranged adjacent to each other in a horizontal line shape having a width of two pixels of the display 31. Further, in the present example, the left-eye optical region 57L and the right-eye optical region 59R coincide (substantially coincide) with a pixel regions A (pixel regions A1 and A2) of a horizontal line shape having a width of two pixels in the display 31.

The polarizing optical member 15 converts a target light flux emitted from the display 31 and passing through the left-eye optical region 57L and a target light flux emitted from the display 31 and passing through the right-eye optical region 59R into target light fluxs having polarization axes orthogonal to each other. For example, in the present example, a target light flux emitted from the display 31 and passing through the left-eye optical region 57L is converted into linearly polarized light having a polarization axis at 45 degrees, and a target light flux passing through the right-eye optical region 59R is converted into linearly polarized light having a polarization axis at 135 degrees.

For example, polarizing lenses 54L and 54R, which will be described later, included in the eye refractive power measurement unit 40 have a polarization axis at 45 degrees and a polarization axis at 135 degrees, respectively. Therefore, when the subject eye observes a visual target through the polarizing lenses 54L and 54R, only the target light flux from the left-eye optical region 57L passes through the polarizing lens 54L and reaches the left eye, and only the target light flux from the right-eye optical region 59R passes through the polarizing lens 54R and reaches the right eye. Accordingly, it is possible to separate the examination visual target for the left eye and the right eye, and different examination visual targets are presented to the left eye and the right eye.

The flat mirror 32 reflects the target light flux from the display 31 and guides the target light flux to the concave mirror 33. The flat mirror 32 reflects the target light flux from the display 31 and guides the target light flux to the subject eye E. For example, the flat mirror 32 is disposed such that a distance (presenting distance) from the subject eye E to the display 31 is optically 40 cm at the time of the near vision examination of the subject eye E. Instead of the flat mirror 32, a reflection member such as a prism, a beam splitter, or a half mirror may be used.

The concave mirror 33 reflects the target light flux from the display 31 and guides the target light flux to the flat mirror 32. For example, the concave mirror 33 is disposed such that the distance (presenting distance) from the subject eye E to the display 31 is optically 5 m at the time of the distance vision examination of the subject eye E. Instead of the concave mirror 33, a reflection member such as an aspherical mirror or a free curved surface mirror may be used. A lens or the like may be used instead of the concave mirror 33.

The distance-near switching unit 34 switches the arrangement of the display 31 between the distance vision examination and the near vision examination of the subject eye E. For example, the distance-near switching unit 34 moves the holding unit by being driven by a drive unit (such as a motor) (not shown), thereby moving the display 31 held by the holding unit. Accordingly, the distant vision arrangement and the near vision arrangement of the display 31 are switched.

For example, at the time of the distance vision examination for the subject eye E, a display screen of the display 31 is directed to a back surface of the housing 1 (see FIG. 2A). The target light flux from the display 31 passes through an optical axis L1 and enters the flat mirror 32, and is reflected by the flat mirror 32 in a direction of an optical axis L2. In addition, the target light flux passes through the optical axis L2 and enters the concave mirror 33, and is reflected by the concave mirror 33 in a direction of an optical axis L3. Further, the target light flux passes through the optical axis L3 and enters the flat mirror 32, and is reflected by the flat mirror 32 in a direction of an optical axis L4. Accordingly, the target light flux emitted to the outside of the housing 1 is projected onto the subject eye E passing through each optical member inside the housing 1.

For example, at the time of the near vision examination for the subject eye E, a display screen of the display 31 is directed to an upper surface of the housing 1 (see FIG. 2B). The target light flux from the display 31 passes through the optical axis L3 and enters the flat mirror 32, and is reflected by the flat mirror 32 in the direction of the optical axis L4. Accordingly, the target light flux emitted to the outside of the housing 1 is projected onto the subject eye E passing through each optical member inside the housing 1.

### <Eye Refractive Power Measurement Unit (Correction Optical System)>

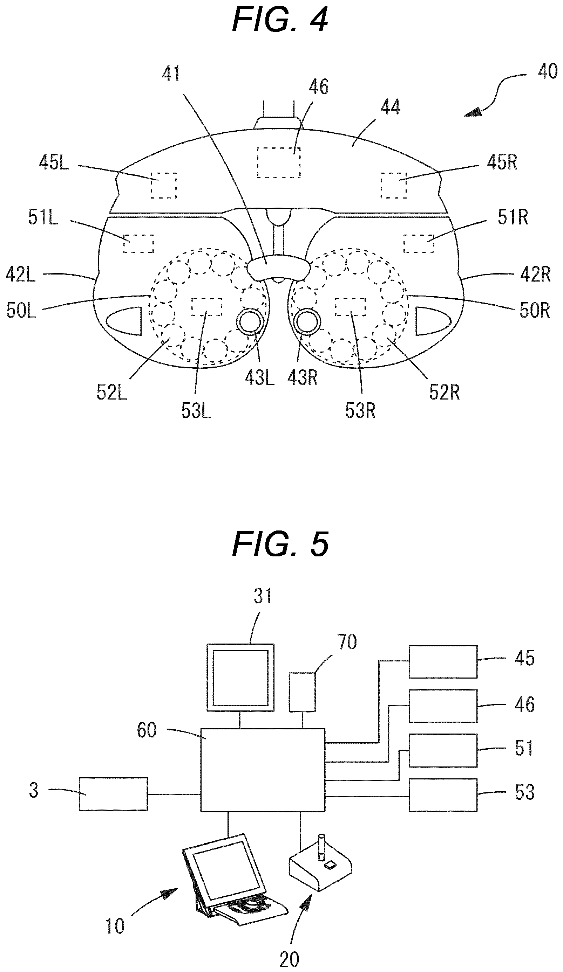

FIG. 4 is a schematic diagram of the eye refractive power measurement unit 40. The eye refractive power measurement unit 40 subjectively measures the refractive power of the subject eye E. The eye refractive power measurement unit 40 is used as a correction optical system. The correction optical system is arranged in an optical path of the light projection optical system 30 to change optical characteristic of the target light flux. For example, the eye refractive power measurement unit 40 includes a forehead rest 41, a lens unit 42, the examination window 43, a moving unit 44, and the like.

The forehead rest 41 comes into contact with the head of the examinee to fix the subject eye E at a predetermined examination position and keep a distance from the subject eye E to the examination window 43 constant. The lens unit 42 includes a left lens unit 42L and a right lens unit 42R, as a pair. The lens unit 42 includes the examination window 43 (a left examination window 43L and a right examination window 43R).

The moving unit 44 adjusts an interval between the left lens unit 42L and the right lens unit 42R and a convergence angle (inward angle) of the left lens unit 42L and the right lens unit 42R. For example, the moving unit 44 adjusts the interval between the left lens unit 42L and the right lens unit 42R by being driven by a drive unit 45 (a left drive unit 45L and a right drive unit 45R). For example, the moving unit 44 adjusts the convergence angle of the left lens unit 42L and the right lens unit 42R by being driven by a drive unit 46. For a detailed configuration of the moving unit 44, for example, refer to JP2004-329345A.

The lens unit 42 includes the lens disk 50 therein. The lens disk 50 includes a left lens disk 50L and a right lens disk 50R, as a pair. The lens disk 50 is rotated by driving of the drive unit 51 (a left drive unit 51L and a right drive unit 51R). In the lens disk 50, an aperture (or a 0D lens) and a plurality of optical elements 52 (a left optical element 52L and a right optical element 52R) are arranged on the same circumference. These optical elements are rotated by driving of the drive unit 53 (a left drive unit 53L and a right drive unit 53R). Thus, the desired optical element 52 is switched into a position at a desired angle in the examination window 43.

The lens disk 50 includes one lens disk or a plurality of lens disks. For example, a spherical lens disk, a cylindrical lens disk, an auxiliary lens disk, or the like may be provided. As an example, the spherical lens disk may include a plurality of spherical lenses having different spherical powers (spherical refractive powers). As an example, the cylindrical lens disk may include a plurality of cylindrical lenses having different cylindrical powers (cylindrical refractive powers). In addition, as an example, the auxiliary lens disk may include a shielding plate, a polarizing lens (polarizing lenses 54L and 54R), a red filter or a green filter, a dispersion prism, a Maddox lens, a rotary prism, a cross cylinder lens, an autocross cylinder lens, an alignment lens, and the like. The drive unit 51 and the drive unit 53 may be provided for each lens disk.

The eye refractive power measurement unit 40 may be any unit capable of changing the optical characteristic of the target light flux. For example, as in the present example, the eye refractive power measurement unit is configured to control the optical element. Further, for example, the wavefront modulating element may be controlled.

### <Control Unit>

FIG. 5 is a schematic diagram of a control system of the subjective optometry device 100. For example, the control unit 60 includes a CPU (a processor), a RAM, a ROM, and the like. For example, the CPU controls each member in the subjective optometry device 100. For example, the RAM temporarily stores various types of information. For example, the ROM stores various programs for controlling the operation of the subjective optometry device 100, examination visual target data, and the like. The control unit 60 may be implemented by a plurality of control units (that is, a plurality of processors).

The control unit 60 is connected to the speaker 3, the display 31, the examiner controller 10, the examinee controller 20, a non-volatile memory 70 (hereinafter, memory 70), and the like. The control unit 60 is connected to the drive unit of the holding unit 4, the drive unit of the distance-near switching unit 34, the drive unit of the eye refractive power measurement unit 40 (drive units 45, 46, 51, 53), and the like.

The memory 70 is a non-transitory storage medium capable of storing storage contents even when a supply of power is cut off. For example, a hard disk drive, a flash ROM, a USB memory, or the like can be used as the memory 70.

### <Control Operation>

A control operation in the first example of the subjective optometry device 100 having the above-described configuration will be described.

The subjective optometry device 100 in the present example can set two self-optometry programs for automatically proceeding with a plurality of examination items for a subject eye based on a response input by the examinee. For example, these two self-optometry programs have different combinations of a plurality of examination items.

For example, one self-optometry program (hereinafter, the first self-optometry program) is a program including, as the plurality of examination items, examination items for examining the refractive power of the subject eye. For example, the examination item for examining the refractive power of the subject eye is a general examination item. As an example, the item is executed when making glasses. For example, the other self-optometry program (second self-optometry program) is a program including an examination item for examining the binocular vision function of the subject eye in addition to the examination item for examining the refractive power of the subject eye. For example, the examination item for examining the binocular vision function of the subject eye is an examination item performed when it is desired to grasp the optical characteristics of the subject eye. The first self-optometry program and the second self-optometry program are stored in the memory 70 in advance.

In addition, the subjective optometry device 100 according to the present example is initially set with the first self-optometry program, and is configured to advance the subjective examination according to the procedure of the first self-optometry program. However, for example, the first self-optometry program and the second self-optometry program can be appropriately switched and set according to the type of glasses desired by the examinee, the examination status of the subject eye (for example, experience of an examiner, optical characteristics of the subject eye, and the like), the operation status (for example, busy times and schedule) of a facility or a store in which the subjective optometry system is installed, or the like.

### <Subjective Examination based on First Self-optometry Program>

Hereinafter, the subjective examination according to the procedure of the first self-optometry program will be described. The examiner uses the self-optometry assistance screen displayed on the monitor 12 of the examiner controller 10 to prepares for the subjective examination according to the procedure of the first self-optometry program, input a start signal, initialize an examination result, and the like.

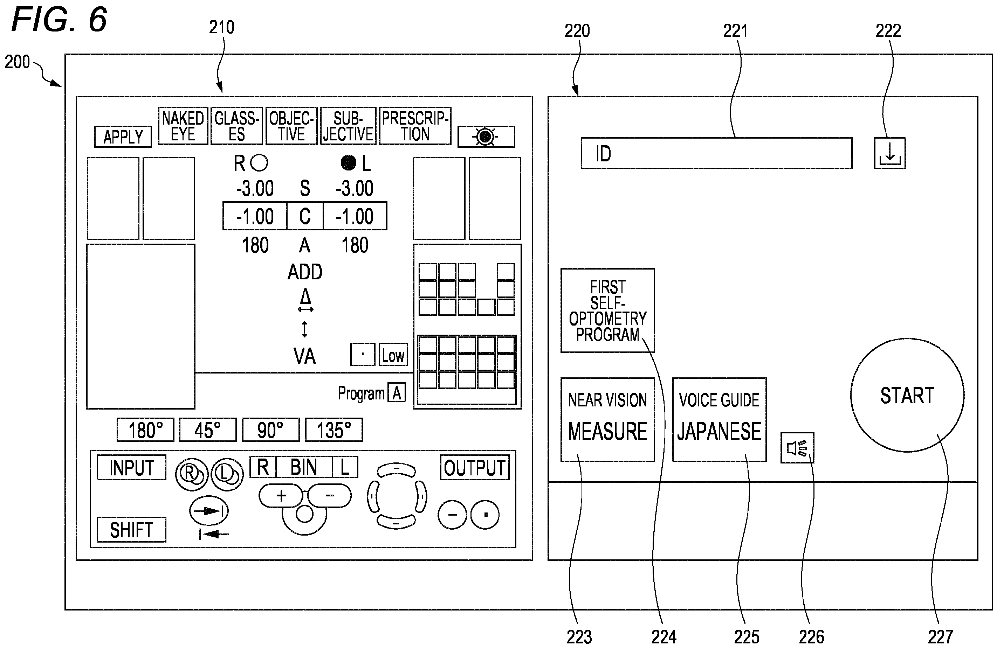

FIG. 6 is an example of a self-optometry assistance screen 200 when the first self-optometry program is set. For example, the self-optometry assistance screen 200 includes an operation image region 210, an optometry program setting region 220, and the like.

The operation image region 210 displays an operation image including information on the optical characteristic of a target light flux emitted from the display 31 toward the subject eye. For example, in the operation image, a value related to the optical characteristic of the target light flux is displayed for each type of optical characteristic. The examiner can specify values of a plurality of types of optical characteristics (spherical power, cylindrical power, astigmatic axis angle, and the like) by operating various buttons, values, and the like on the operation image region 210 via a touch panel or the switch unit 11. When the self-optometry is appropriately executed, the correction value of the optical characteristic displayed in the operation image becomes a measurement value of the optical characteristic of the subject eye.

The optometry program setting region 220 includes an ID button 221, an initial value input button 222, a near vision measurement selection button 223, a self-optometry program switching button 224, a voice guide selection button 225, a volume test button 226, a start button 227, and the like. For example, the ID button 221 is a button for inputting the ID of the examinee. For example, the initial value input button 222 is a button for acquiring an initial correction value (initial value) when starting the self-optometry for the subject eye. As an example, the initial value may be the objective eye refractive power of the subject eye measured by an objective eye refractive power measurement device, lens information of the glasses measured by a glass lens measurement device, or the like. For example, the near vision measurement selection button 223 is a button for selecting whether to perform near vision measurement in the subjective examination. For example, the self-optometry program switching button 224 is a button for switching between the first self-optometry program and the second self-optometry program. The name of a currently selected self-optometry program is displayed superimposed on the self-optometry program switching button 224. For example, the voice guide selection button 225 is a button for changing the language of the voice guide. For example, the volume test button 226 is a button for playing a test voice to check the volume of the voice guide. For example, the start button 227 is a button for starting the self-optometry.

### <Setting of Initial Value>

First, the examiner acquires the objective eye refractive power of the subject eye in order to correct the subject eye to a predetermined correction value (initial value). For example, after inputting the examinee ID using the ID button 221, the examiner presses the initial value input button 222. For example, the control unit 60 outputs an operation signal to the objective eye refractive power measurement device (not illustrated) so as to transmit the objective eye refractive power associated with the examinee ID, based on the input signal from the initial value input button 222. For example, when receiving the examinee ID from the subjective optometry device 100, the objective eye refractive power measurement device calls up the objective eye refractive power corresponding to the examinee ID from the memory and transmits the objective eye refractive power to the subjective optometry device 100. The control unit 60 of the subjective optometry device 100 receives the objective eye refractive power transmitted from the objective eye refractive power measurement device. Accordingly, the objective eye refractive power of the examinee can be acquired.

For example, the control unit 60 sets the objective eye refractive power as an initial value of the subjective examination. For example, the control unit 60 controls the eye refractive power measurement unit 40 to place the optical element 52 having a predetermined spherical power and the optical element 52 having a cylindrical power in the examination window 43. Further, for example, the control unit 60 places the optical element 52 having a predetermined cylindrical power in the examination window 43 at a predetermined astigmatic axis angle. Accordingly, the subject eye E is corrected to the initial value.

### <Subjective Examination>

After correcting the subject eye E to the initial value, the examiner checks the self-optometry program switching button 224 of the self-optometry assistance screen 200, checks the currently set self-optometry program, and presses the start button 227. The control unit 60 starts the self-optometry of the subject eye E based on the input signal from the start button 227. For example, in the first self-optometry program, the spherical examination, the astigmatic axis examination, the cylindrical examination, and a visual acuity examination are performed as examination items for examining the refractive power of the subject eye. For example, the control unit 60 advances these examinations in sequence while changing the correction value of the subject eye, the type of the examination visual target to be presented to the subject eye E, the visual acuity value, and the like.

### <Spherical Examination (Step S1)>

In the present example, a red-green test is performed to adjust the spherical correction value for correcting the subject eye. In the red-green test, it is possible to determine whether a state of the subject eye corrected with a desired spherical correction value corresponds to myopia, emmetropia, or hyperopia. For example, it is determined as myopic if a red visual target is clearly visible, it is determined as hyperopic if a green visual target is clearly visible, and it is determined as emmetropia if both red and green are clearly visible. Note that, for example, the hyperopia is considered to be overcorrected, and the spherical correction is reduced until emmetropia (or myopia) is achieved.

At the start of the red-green test, the control unit 60 causes the speaker 3 to generate a voice guide indicating how to operate the examinee controller 20 in the red-green test. As an example, a voice guide may be generated to indicate that tilt the response lever 21 to the left when, of the red visual target and the green visual target, a clearly visible visual target is red, tilt the response lever 21 to the right when the clearly visible target is green, and press the response button 22 when they are approximately the same.

Next, the control unit 60 advances the examination and displays a red-green visual target on the display 31. For example, the red of the red-green visual target is displayed on the left side and the green is displayed on right side. In addition, the control unit 60 generates a voice guide for asking the examinee which color of the visual target is clearly visible to the examinee. The examinee checks the visual target, and tilts the response lever 21 to the left or right, or presses the response button 22.

The control unit 60 increases or decreases the spherical power by one step according to a tilt direction of the response lever 21 when receiving a response signal from the response lever 21 in response to the examinee selecting either the red or green visual target. In addition, the control unit 60 generates a voice guide asking about the color of the visual target again. For example, the control unit 60 repeats such control to automatically advance the examination.

For example, when the control unit 60 receives a response signal from the response button 22 in response to the examinee determining that the red and green visual targets are substantially the same, the control unit 60 ends the red-green test. The spherical correction value by which the subject eye is corrected at the end time of the examination is used as the spherical correction value at the start of the visual acuity examination.

### <Astigmatic Axis Examination (Step S2)>

Subsequently, in the present example, a cross cylinder test is performed to adjust an astigmatic axis angle for correcting the subject eye. Here, it is possible to determine whether the astigmatic axis angle of the subject eye coincides with the astigmatic axis angle desired for correcting the subject eye. For example, if there is a difference between appearances of the two point cloud visual targets, the respective angles are determined to be inconsistent, and if the appearances of the two point cloud visual targets are substantially the same, the respective angles are determined to be consistent.

At the start of the cross cylinder test, the control unit 60 causes the speaker 3 to generate a voice guide indicating how to operate the examinee controller 20 in the cross cylinder test. As an example, a voice guide is generated to indicate that tilt the response lever 21 to a direction of the clearly visible point group visual target, and press the response button 22 if both groups are the same. The control unit 60 repeatedly changes the astigmatic axis angle until a response signal is received from the response button 22, and ends the cross cylinder test when a response signal is received from the response button 22. The astigmatic axis angle by which the subject eye is corrected at the end time of the examination is used as the astigmatic axis angle at the start of the visual acuity examination.

### <Cylindrical Examination (Step S3)>

Subsequently, in the present example, the cross cylinder test is performed again in order to adjust a cylindrical correction value for correcting the subject eye. Here, it is possible to determine whether a state in which the subject eye is corrected with a desired cylindrical power is appropriate. For example, if there is a difference between the appearances of the two point cloud visual targets, it is determined to be inappropriate, and if the appearances of the two point cloud visual targets are substantially the same, it is determined to be appropriate. Similarly, the control unit 60 generates the voice guide, repeatedly changes the cylindrical correction value until a response signal is received from the response button 22, and ends the cross cylinder test when a response signal is received from the response button 22. The cylindrical correction value by which the subject eye is corrected at the end time of the examination is used as the cylindrical correction value at the start of the visual acuity examination.

### <Spherical Examination (Step S4)>

Subsequently, in the present example, the red-green test is performed again in order to adjust the spherical correction value for correcting the subject eye. Here, it is checked whether the adjustment by the subject eye is functioning. As an example, the spherical correction value is the same at the end of step S1 and at the start of step S4, but the appearance of the red-green visual target changes when the adjustment is functioning. Since the red-green test in step S4 is basically the same as the red-green test in step S1, the description thereof will be omitted.

### <Visual Acuity Examination (Step S5)>

Subsequently, a corrected visual acuity test (VA test) is performed to acquire the highest visual acuity value in a state where the subject eye is corrected with the predetermined correction value adjusted in step S1 to step S4.

At the start of the corrected visual acuity test, the control unit 60 causes the speaker 3 to generate a voice guide indicating how to operate the examinee controller 20 in the corrected visual acuity test. As an example, a voice guide is generated to indicate that tilt the response lever 21 to a direction of the gap of the Landolt ring visual target, and press the response button 22 if the direction of the gap is unclear. The control unit 60 increases or decreases the visual acuity value by one step according to a tilt direction of the response lever 21 when receiving a response signal from the response lever 21 in response to the examinee selecting the direction of the gap. The control unit 60 repeatedly changes the direction of the gap until a response signal is received from the response button 22, and ends the corrected visual acuity test when a response signal is received from the response button 22. For example, the visual acuity value of the Landolt ring visual target that the examinee last responseed correctly is taken as the highest visual acuity value.

### <Initialization>

In the present example, in the self-optometry using the subjective optometry device 100, each examination in step S1 to step S4 described above is performed on the right eye and then similarly performed on the left eye. The control unit 60 outputs examination results of the right eye and the left eye (for example, measurement values such as a spherical correction value, a cylindrical correction value, and an astigmatic axis angle). For example, the control unit 60 may display the measurement values on the self-optometry assistance screen 200, print out the measurement values, or output the measurement values to the outside of the device by wireless communication or wired communication.

The examiner checks the examination results of the subject eye and presses an examination end button (not shown) for ending the self-optometry of the subject eye. The control unit 60 clears the examination results displayed on the self-optometry assistance screen 200 in response to an input signal from the examination end button. This completes the initialization of the subjective optometry device 100.

In the present example, when the first self-optometry program is set as described above, the examination items (spherical examination, astigmatic axis examination, cylindrical examination, and visual acuity examination) for examining the refractive power of the subject eye are automatically proceeded. For example, by setting the first self-optometry program and proceeding with a limited number of predetermined examination items, even an examiner with little experience can easily handle the examination. In addition, since the time required for the self-optometry is shortened, it is possible to smoothly perform the examination in consideration of the congestion situation of the facility or the store in which the device is installed.

### <Subjective Examination based on Second Self-optometry Program>

In the present example, the first self-optometry program is initially set, but it is possible to switch to the second self-optometry program depending on the type of glasses desired by the examinee and various circumstances when examining the subject eye. As an example, it is possible to switch the self-optometry program as appropriate, for example in a case where the position or the vision of the line of sight of the examinee requires finely adjusted, that is, a so-called custom lens is required, or in a case where a facility or a store is not busy and more attentive care can be provided.

Hereinafter, the subjective examination according to the procedure of the second self-optometry program will be described. First, the examiner switches the self-optometry program using the self-optometry assistance screen displayed on the monitor 12 of the examiner controller 10.

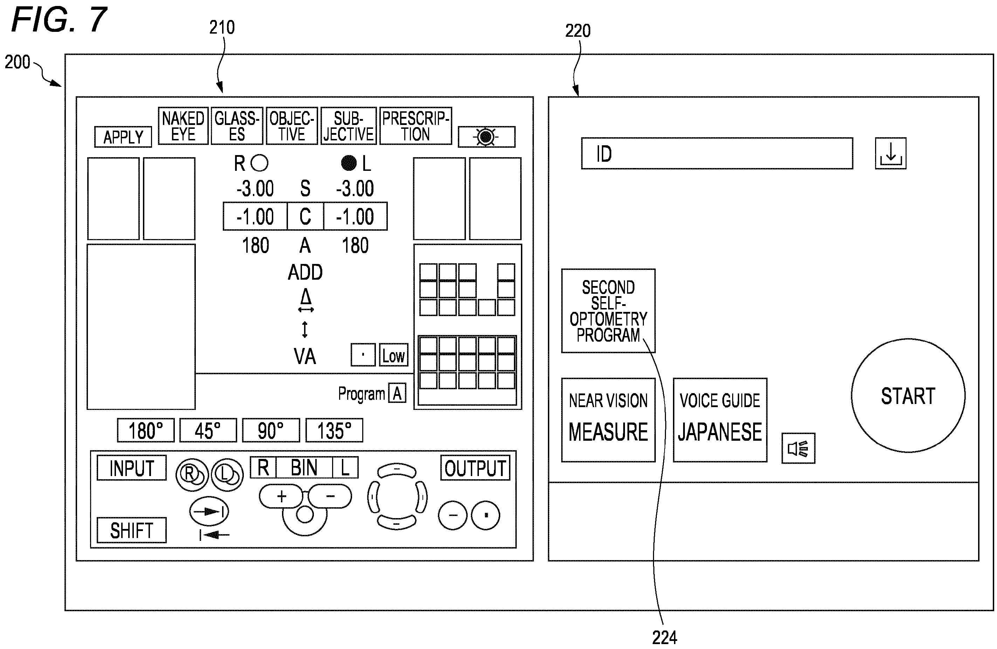

FIG. 7 is an example of the self-optometry assistance screen 200 when the second self-optometry program is set. For example, the examiner changes the setting from the first self-optometry program to the second self-optometry program by pressing the self-optometry program switching button 224. The control unit 60 changes the superimposed display of the name of the self-optometry program in the self-optometry program switching button 224. The control unit 60 calls up the second self-optometry program from the memory 70 and applies the second self-optometry program.

In addition, the examiner prepares the subjective examination (for example, sets an initial value for correcting the subject eye E) according to the procedure of the second self-optometry program using the self-optometry assistance screen 200, and starts the self-optometry. For example, in the second self-optometry program, in addition to an examination item for examining the refractive power of the subject eye, a binocular accommodation balance examination for examining the balance of accommodation of the subject eye is performed as an examination item for examining the binocular vision function of the subject eye. For example, the control unit 60 advances these examinations in sequence while changing the correction value of the subject eye, the type of the examination visual target to be presented to the subject eye E, the visual acuity value, and the like.

For example, similarly to the first self-optometry program, in the second self-optometry program, the process also proceeds in order from the spherical examination (step S1) to the visual acuity examination (step S5) (description thereof will be omitted). For example, once the spherical correction value, the cylindrical correction value, the astigmatic axis angle, and the like are obtained for each of the right eye and the left eye, the process proceeds to the binocular accommodation balance examination.

### <Binocular Accommodation Balance Examination (Step S6)>

In the present example, a polarized red-green test is performed to adjust the balance between the appearances for the right eye and the left eye. In the polarized red-green test, it is possible to determine whether the adjustment for the right eye and the left eye is equivalent. For example, when there is a difference between how the red visual target and the green visual target in the upper row appear and how the red visual target and the green visual target in the lower row appear in the polarized red-green visual target, it is determined that the balance is not achieved.

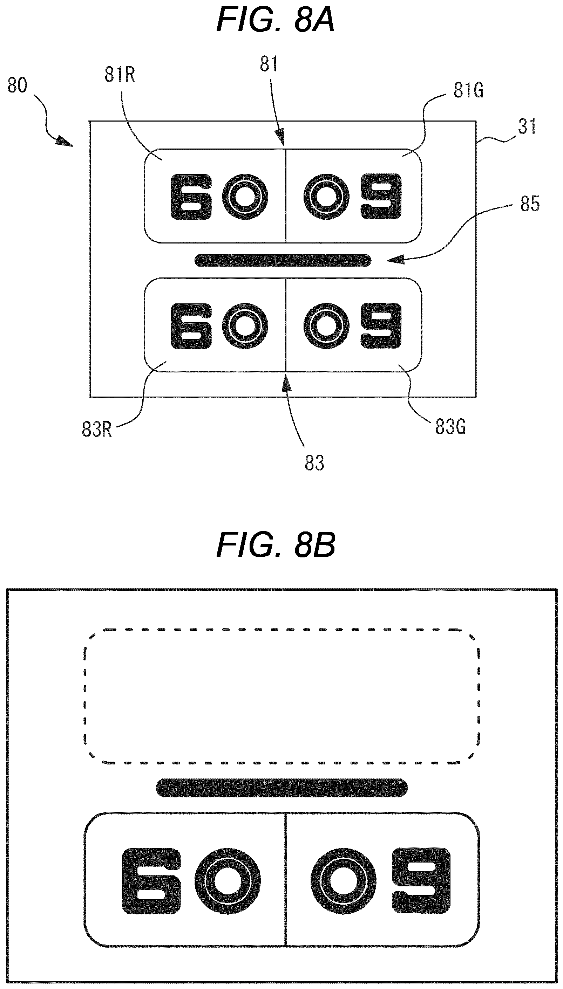

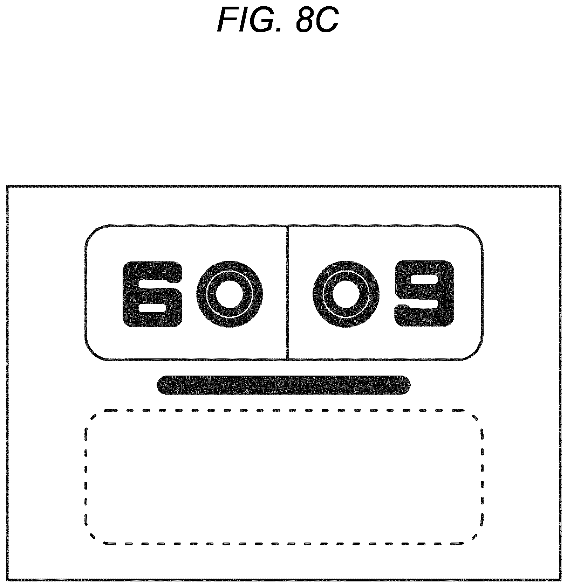

FIG. 8 is a diagram illustrating a polarized red-green visual target 80. FIG. 8A illustrates how the visual target appears to both eyes. FIG. 8B illustrates how the visual target appears to the left eye. FIG. 8C illustrates how the visual target appears to the right eye. For example, the polarized red-green visual target 80 includes a left eye polarized red-green visual target 83 (hereinafter, referred to as a left polarized R/G visual target 83), a right eye polarized red-green visual target 81 (hereinafter, referred to as a right polarized R/G visual target 81), and a fusion visual target 85. For example, the left polarized R/G visual target 83 includes a red visual target 83R disposed on the left side and a green visual target 83G disposed on the right side. For example, the right polarized R/G visual target 81 includes a red visual target 81R disposed on the left side and a green visual target 81G disposed on the right side. For example, the fusion visual target 85 is disposed between the right polarized R/G visual target 81 and the left polarized R/G visual target 83.

At the start of the polarized red-green test, the control unit 60 causes the speaker 3 to generate a voice guide indicating how to operate the examinee controller 20 in the polarized red-green test. As an example, a voice guide may be generated to indicate that tilt the response lever 21 to the left when the green visual target can be clearly visually recognized in the upper row (right eye) and the red visual target can be clearly visually recognized in the lower row (left eye). Further, a voice guide may be generated to indicate that tilt the response lever 21 to the right when the red visual target can be clearly visually recognized in the upper row and the green visual target can be clearly visually recognized in the lower row. Further, a voice guide may be generated to indicate that press the response button 22 when the green visual targets in the upper and lower rows can be clearly visually recognized, when the red visual targets in the upper and lower rows can be clearly visually recognized, or when all the visual targets in the upper and lower rows can be clearly visually recognized to the same extent.

Next, the control unit 60 advances the examination and displays a polarized red-green visual target on the display 31. The control unit 60 disposes polarizing lenses 30L and 30R in the examination window 43 of the eye refractive power measurement unit 40. The control unit 60 increases or decreases the spherical correction value by one step according to a tilt direction of the response lever 21 when receiving a response signal from the response lever 21 by the examinee. For example, the control unit 60 repeatedly adjusts the spherical correction value until a response signal is received from the response button 22, and ends the polarized red-green test when a response signal is received from the response button 22.

When the binocular accommodation balance examination of the subject eye E is completed, the control unit 60 outputs examination results such as a spherical correction value, a cylindrical correction value, and an astigmatic axis angle of the subject eye E. In addition, the control unit 60 clears the examination results displayed on the self-optometry assistance screen 200 in response to an input signal from an examination end button (not illustrated) by the operation of the examiner, and completes the initialization.

In the present example, when the second self-optometry program is set as described above, the binocular vision function examination for the subject eye E is automatically performed following the examination items (spherical examination, astigmatic axis examination, cylindrical examination, and visual acuity examination) for examining the refractive power of the subject eye. Of course, the binocular vision function examination may be an examination item different from the binocular adjustment balance test described above, or may be an examination item different from the binocular adjustment balance test in addition to the binocular adjustment balance test. As a result, a more detailed examination result of the subject eye can be acquired by the self-optometry.

### <Second Example>

A second example of the subjective optometry system according to the present embodiment will be described. Here, a case where the self-optometry program applied to the subjective optometry device 100 is automatically switched by setting a schedule associated with a predetermined period will be described as an example. Since the configuration (for example, the appearance of the device, the light projection optical system, the control unit, and the like) similar to that of the first example is the same as that of the first example, detailed description thereof will be omitted.

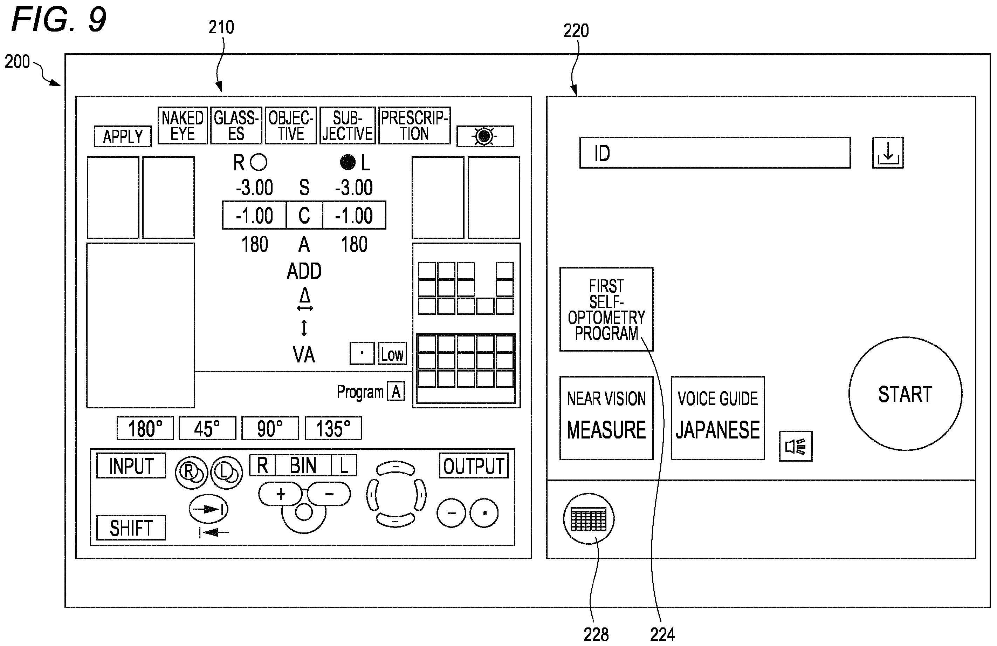

FIG. 9 is an example of a self-optometry assistance screen in the second example. The self-optometry assistance screen 200 may be provided with a separate schedule setting button 228. For example, the schedule setting button 228 is a button for displaying a schedule setting screen. For example, the schedule setting screen is a screen for setting the self-optometry program based on calendar information.

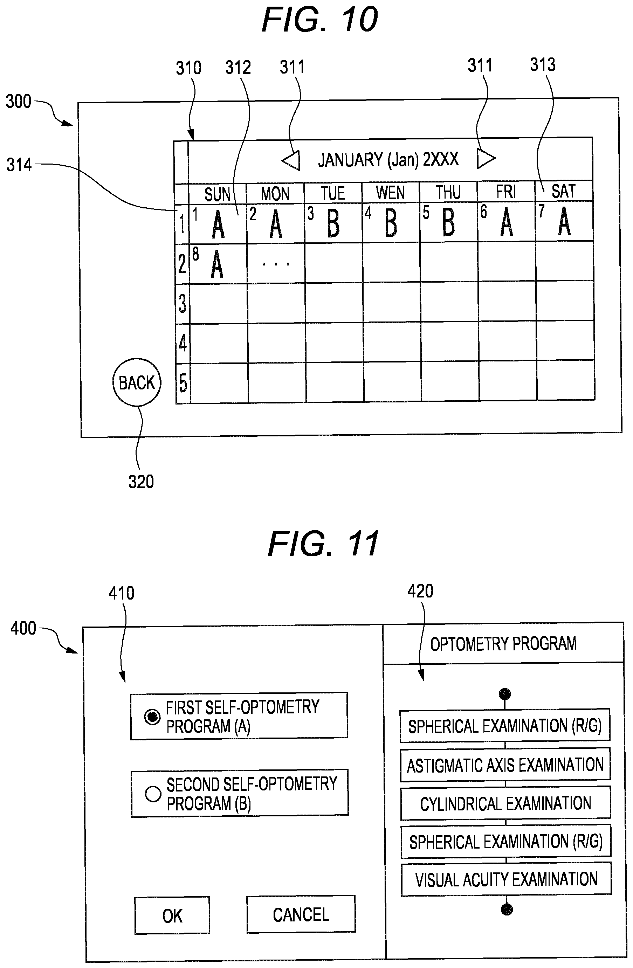

FIG. 10 is an example of a schedule setting screen 300. For example, the schedule setting screen 300 includes a calendar screen 310, a month switching button 311, a date button 312, a day-of-week batch switching button 313, a weekly batch switching button 314, and a screen switching button 320. For example, the calendar screen 310 is a screen for setting and checking the self-optometry program for each date. For example, the month switching button 311 is a button for switching the calendar (month) of the calendar screen 310. For example, the date button 312 is a button for setting the self-optometry program for a desired date. For example, the day-of-week batch switching button 313 is a button for collectively setting the self-optometry program on a day-by-day basis. For example, the weekly batch switching button 314 is a button for collectively setting the self-optometry program on a weekly basis. For example, the screen switching button 320 is a button for returning to the self-optometry assistance screen 200 (see FIG. 9).

### <Control Operation>

A control operation in the second example of the subjective optometry device 100 having the above-described configuration will be described. The subjective optometry device 100 according to the present example automatically switches the first self-optometry program and the second self-optometry program according to an optometry schedule set in advance. For example, it is possible to smoothly perform the self-optometry on the subject eye by setting the optometry schedule according to, for example, an operation status (for example, busy times and schedule) of a facility or a store in which the device is installed.

### <Setting of Optometry Schedule>

First, the examiner sets an optometry schedule in which the first self-optometry program and the second self-optometry program are associated with a predetermined period. For example, the examiner operates the examiner controller 10 to press the schedule setting button 228 of the self-optometry assistance screen 200. The control unit 60 displays the schedule setting screen 300 based on an input signal from the schedule setting button 228. Further, for example, the examiner presses any one of the date button 312, the day-of-week batch switching button 313, and the weekly batch switching button 314 from the calendar screen 310 of the schedule setting screen 300. Here, a case where the date button 312 is selected will be described as an example. The control unit 60 displays a self-optometry program selection screen based on an input signal from the date button 312. For example, the self-optometry program selection screen is a screen for setting a desired self-optometry program on a date designated by the date button 312.

FIG. 11 is an example of a self-optometry program selection screen 400. For example, the self-optometry program selection screen 400 may include a self-optometry program selection field 410, a self-optometry program display field 420, and the like. For example, the self-optometry program selection field 410 includes a radio button for selecting either the first self-optometry program or the second self-optometry program. For example, the self-optometry program display field 420 displays an order of proceeding with the examination items for the self-optometry program selected by the radio button in the self-optometry program selection field 410.

For example, the examiner checks the examination content of each self-optometry program using the self-optometry program display field 420, and selects a desired self-optometry program using the radio button in the self-optometry program selection field 410. For example, the control unit 60 displays the schedule setting screen 300 (see FIG. 10) based on an input signal from the self-optometry program selection screen 400, and displays a code or the like (in this example, "A" in the case of the first self-optometry program and "B" in the case of the second self-optometry program) that allows recognition of the self-optometry program selected by the examiner on the date button 312 in a superimposed manner.

For example, the examiner operates the date buttons 312 one by one to set the first self-optometry program and the second self-optometry program for a period such as one week, one month, or half a year. For example, the first self-optometry program "A" may be initially set in advance in each date button 312, and the second self-optometry program "B" may be selected only for a predetermined date in the date button 312. In addition, for example, the setting in a predetermined date button 312 may be configured to be duplicated on a different date, or may be configured to be collectively set for several days. In addition, for example, the setting of the first self-optometry program or the second self-optometry program may be set by a time table or the like. That is, a time zone in which the first self-optometry program or the second self-optometry program is applied may be set. As an example, the setting of the self-optometry program may be changed in time units such as morning and afternoon.

For example, the examiner presses the screen switching button 320 to return to the self-optometry assistance screen 200. The control unit 60 stores the date of the date button 312 and the self-optometry program selected by the examiner in association with each other in the memory 70 according to the operation signal from the screen switching button 320. Accordingly, for example, an optometry schedule is created.

### <Switching of Self-Optometry Program>

The control unit 60 acquires the current date and time by a clocking mechanism (for example, a calendar timer) (not illustrated) provided in the subjective optometry device 100. In addition, when a predetermined time (for example, 0:00:00 a.m.) for changing the self-optometry program arrives, the control unit 60 calls up and applies the designated self-optometry program from the memory 70 according to the optometry schedule, and automatically switches the self-optometry program. For example, the control unit 60 may overwrite the setting of the designated self-optometry program only at a predetermined time on the day when the first self-optometry program and the second self-optometry program are switched on the optometry schedule. Further, for example, the control unit 60 may cancel the setting of the current self-optometry program and reflect the setting of the designated self-optometry program every time a predetermined time is reached every day regardless of whether the first self-optometry program and the second self-optometry program are switched on the optometry schedule.

In the present example, as described above, it is possible to set the optometry schedule associated with each self-optometry program in advance. For example, by grasping the work shift of the examiner, the operation status of the facility, the busy times, and the like and setting the optometry schedule that takes these into consideration, it is possible to easily set an appropriate self-optometry program and smoothly perform the examination.

As described above, for example, a subjective optometry system according to the present example includes: a target presentation unit configured to present an examination visual target to a subject eye; a correction unit configured to change an optical characteristic of a target light flux emitted from the target presentation unit; a self-optometry program setting unit configured to set either a first self-optometry program that automatically proceeds with a plurality of examination items for the subject eye based on a response input by an examinee, or a second self-optometry program that automatically proceeds with a plurality of examination items for the subject eye based on a response input by the examinee, the second self-optometry program having a different examination condition from the first self-optometry program; and a control unit configured to control, based on the first self-optometry program or the second self-optometry program set by the self-optometry program setting unit, at least one of the target presentation unit and the correction unit to advance an optometry according to a procedure of the self-optometry program. With such a configuration, it is possible to easily apply a program according to the type of glasses, the examination status, the operation status, and the like, and to smoothly perform the examination.

Further, for example, in the subjective optometry system according to the present example, the first self-optometry program and the second self-optometry program differ in at least one of the plurality of examination items as the examination condition. Accordingly, for example, the first self-optometry program and the second self-optometry program are appropriately set, and the program of the examination item is executed according to the type of glasses desired by the examinee, the examination status of the subject eye, the operation status of the facility or store in which the device is installed, and the like, so that the examination can be smoothly advanced.

Further, for example, in the subjective optometry system according to the present example, the first self-optometry program includes, as the plurality of examination items, an examination item for examining a refractive power of the subject eye, and the second self-optometry program includes, as the plurality of examination items, an examination item for examining a binocular vision function of the subject eye in addition to the examination item for examining the refractive power of the subject eye. For example, the examination item for examining the refractive power of the subject eye is a general examination item. Therefore, by setting the first self-optometry program, it is possible to efficiently cope with many examinees. In addition, for example, the examination item for examining the binocular vision function of the subject eye is an examination item performed when it is desired to grasp the optical characteristics of the subject eye. Therefore, by setting the second self-optometry program, it is possible to make glasses that are more optimal for the subject eye by using the examination results of the refractive power and the visual function of the subject eye.

Further, for example, the subjective optometry system according to the present example further includes an optometry schedule setting unit configured to set an optometry schedule in which the first self-optometry program and the second self-optometry program are each associated with a predetermined period, in which the self-optometry program setting unit sets to switch between the first self-optometry program and the second self-optometry program, based on the optometry schedule set by the optometry schedule setting unit. Accordingly, for example, it is possible to set an optometry schedule according to an operation status of a facility or a store in which the subjective optometry system is installed, and since the self-optometry program is automatically switched every predetermined period, it is possible to smoothly perform the examination.

Further, for example, the subjective optometry system according to the present example further includes an operation unit configured to input an operation signal for setting at least one of the first self-optometry program and the second self-optometry program, in which the optometry schedule setting unit sets the optometry schedule in which the first self-optometry program and the second self-optometry program are each associated with a predetermined period, based on the operation signal from the operation unit. For example, an administrator of a facility or a store in which the subjective optometry system is installed or an examiner using the subjective optometry system can set an optimal optometry schedule for each facility or store, and a self-optometry program is automatically applied according to an operation status or the like, so that an examination can be smoothly performed.

### <Modifications>

In the present example, the first self-optometry program and the second self-optometry program have been described with a configuration in which a plurality of examination items are different as the examination conditions, but the present invention is not limited thereto. For example, the first self-optometry program and the second self-optometry program may be configured such that the examination visual targets used in a plurality of examination items are different, as the examination conditions. For example, both the first self-optometry program and the second self-optometry program may be programs that sequentially execute from the spherical examination to the visual acuity examination. Of course, in the second self-optometry program, the binocular accommodation balance examination, an eye position examination, or the like may be further performed. For example, in this case, the examination visual targets used in the visual acuity examination of the first self-optometry program and in the visual acuity examination of the second self-optometry program may be different. As an example, the Landolt ring visual target or the tumbling E visual target may be used. Similarly, different examination visual targets may be set for the spherical examination, the astigmatic axis examination, and the cylindrical examination of the first self-optometry program and the second self-optometry program. Each self-optometry program does not necessarily have different examination visual targets set for all common examination items.

In the subjective optometry system according the present example, the first self-optometry program and the second self-optometry program have different examination visual targets used in the examination item, as the examination condition. For example, in the examination for the subject eye, the type of the examination visual target or the display format may be changed to suit the country or region in which the subjective optometry system is installed, or the number or the type of the examination visual targets may be changed according to the examination status of the subject eye. As in the present example, since a plurality of self-optometry programs having different examination visual targets are provided in advance, the self-optometry program can be switched as necessary, and the examination can be smoothly performed.

In the present example, the configuration in which either the first self-optometry program or the second self-optometry program is manually set has been described as an example, but the present invention is not limited thereto. In the present example, these self-optometry programs may be automatically set. For example, in this case, information of a reservation system or the like that can check the busy status of the facility or store may be used. For example, in this case, examinee information (examinee ID or the like) may be used.

For example, when information of a reservation system or the like is used, the control unit 60 may switch the self-optometry program based on whether the number of examinees waiting for the optometry exceeds a certain number. For example, the control unit 60 may set the first self-optometry program when the number of examinees registered in the reservation system exceeds a certain number. In the present example, by applying the first self-optometry program, only general refraction examination items are performed, and thus the self-optometry can be efficiently performed even in situations where there are many examinees waiting. Further, for example, the control unit 60 may set the second self-optometry program when the number of examinees registered in the reservation system is less than a certain number. In the present example, the binocular accommodation balance examination or the like is further performed by applying the second self-optometry program, so that it is possible to acquire a more detailed examination result of the subject eye when there are not many examinees waiting.

For example, when the examinee information is used, the control unit 60 may switch the self-optometry program based on data associated with the examinee information such as the type of glasses made last time, the previous examination result, and the presence or absence of a disease. For example, the control unit 60 may set the second self-optometry program when the examinee information includes a history of the execution of the binocular accommodation balance examination in the previous examination.

In the present example, a configuration in which the examination conditions of the first self-optometry program and the second self-optometry program are set in advance and the examination proceeds with predetermined examination items has been described as an example, but the present invention is not limited thereto. For example, at least one of the examination conditions of the first self-optometry program and the second self-optometry program may be changed in advance.

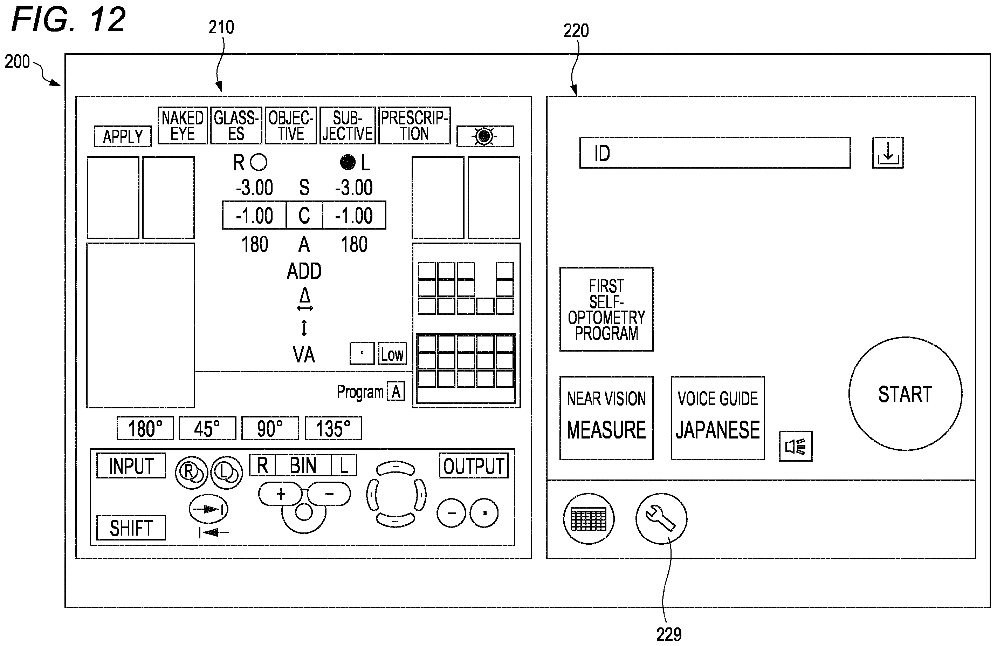

FIG. 12 is an example of the self-optometry assistance screen 200 for editing the examination conditions of the self-optometry program. For example, a self-optometry program editing button 229 may be provided on the self-optometry assistance screen 200. For example, the self-optometry program editing button 229 is a button for displaying a self-optometry program editing screen.

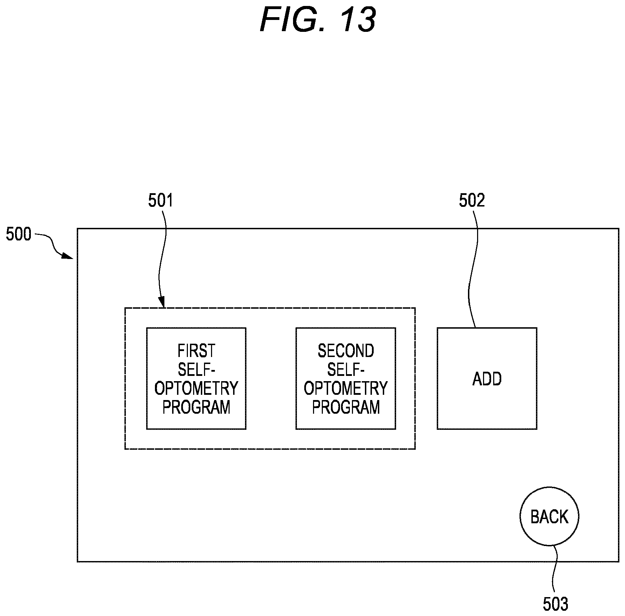

FIG. 13 is an example of a self-optometry program selection screen 500. For example, the self-optometry program selection screen 500 is provided with a self-optometry program selection button 501, a self-optometry program adding button 502, a screen switching button 503, and the like. For example, the self-optometry program selection button 501 is a button for editing the self-optometry program. For example, the self-optometry program adding button 502 is a button for additionally registering the self-optometry program. For example, the screen switching button 503 is a button for returning to the self-optometry assistance screen 200 (see FIG. 12).

For example, when the examiner presses the self-optometry program selection button 501 of the self-optometry program selection screen 500, the control unit 60 displays the self-optometry program editing screen based on the input signal from the self-optometry program selection button 501. For example, the self-optometry program editing screen is a screen for editing the content of the self-optometry program.

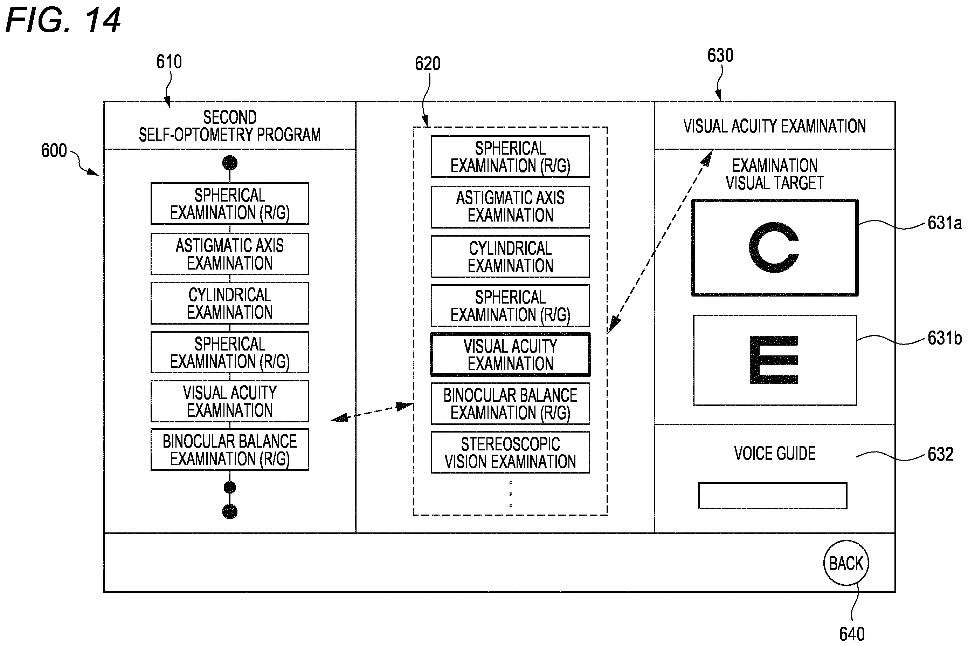

FIG. 14 is an example of a self-optometry program editing screen 600. For example, the self-optometry program editing screen 600 is provided with a self-optometry program sequence field 610, an examination item selection field 620, an examination item editing field 630, a screen switching button 640, and the like. For example, the self-optometry program sequence field 610 enables a change in the sequence of the examination items of the self-optometry program, and the examination items can be added or deleted. For example, the examination item selection field 620 displays examination items that can be added to the self-optometry program. For example, the examination item editing field 630 changes the examination visual target used in the examination item selected in the examination item selection field 620. For example, the voice guide selection field 632 selects the content of the voice guide for the examination item selected in the examination item selection field 620. For example, the screen switching button 640 is a button for returning to the self-optometry program selection screen 500 (see FIG. 13).

For example, the examiner can change whether to execute the examination item included in at least one of the first self-optometry program and the second self-optometry program from the self-optometry assistance screen 200. As an example, here, a case where a stereoscopic vision examination is added to the examination items of the second self-optometry program will be described. The examiner presses the self-optometry program editing button 229 on the self-optometry assistance screen 200. The control unit 60 displays the self-optometry program selection screen 500 based on an input signal from the self-optometry program editing button 229. Further, the examiner presses the self-optometry program selection button 501 on the self-optometry program selection screen 500. For example, a button corresponding to the second self-optometry program is pressed. The control unit 60 displays the self-optometry program editing screen 600 based on an input signal from the self-optometry program selection button 501.

The examiner drags and drops the stereoscopic vision examination selected from the list of examination items displayed in the examination item selection field 620 to any position in the sequence of the examination items in the second self-optometry program displayed in the self-optometry program sequence field 610. For example, drag and drop the stereoscopic vision examination to the position next to the binocular adjustment balance test. When the second self-optometry program is set, the control unit 60 incorporates the stereoscopic vision examination in the sequence of the second self-optometry program so as to execute the stereoscopic vision examination in addition to the spherical examination to the binocular accommodation balance examination described above. For example, as described above, it is possible to change whether to execute the examination item included in each self-optometry program.

Further, for example, the examiner can change the type of the examination visual target used in the examination item included in at least one of the first self-optometry program and the second self-optometry program from the self-optometry assistance screen 200. As an example, here, a case where the type of the examination visual target used in the corrected visual acuity test (VA test) of the second self-optometry program is changed will be described. The examiner operates the self-optometry program editing button 229 and the self-optometry program selection button 501 to call up the self-optometry program editing screen 600.

The examiner selects the corrected visual acuity test (VA test) from the list of examination items displayed in the examination item selection field 620. Based on the input signal from the examination item selection field 620, the control unit 60 displays the type of the examination visual target set in the corrected visual acuity test in the examination item editing field 630. For example, either the Landolt ring visual target 631a or the tumbling E visual target 631b can be set as the type of the examination visual target, and a field corresponding to the examination visual target selected by the examiner may be highlighted. The control unit 60 reflects this in the setting of the second self-optometry program so that the Landolt ring visual target is displayed in the corrected visual acuity test. For example, in this way, the type of the examination visual target used in the examination item included in the self-optometry program can be changed.

The examiner can also add a self-optometry program different from the first self-optometry program and the second self-optometry program by using the self-optometry program adding button 502. The examiner can also change the content of the voice guide for the examination item selected in the examination item selection field 620 from the voice guide selection field 632 of the self-optometry program editing screen 600. For example, at least one of a message to be read by the voice guide, a frequency of repeating the voice guide, and the like may be changed.

As described above, the subjective optometry system according to the present example includes the setting change unit that changes at least one examination condition of the examination condition of the first self-optometry program and the examination condition of the second self-optometry program. For example, depending on the examinee, the examination conditions of the first self-optometry program and the second self-optometry program provided in advance in the subjective optometry system may not be suitable. For example, in such a case, by changing (that is, rewriting) the examination conditions of the self-optometry program so as to achieve the examination conditions desired by the examiner before the start of the self-optometry for the subject eye, the examination can be smoothly performed under the desired examination conditions.

In the present example, the configuration in which the optometry schedule in which either the first self-optometry program or the second self-optometry program is associated with the predetermined period is manually set has been described as an example, but the present invention is not limited thereto. In the present example, such an optometry schedule may be automatically set. For example, the optometry schedule may be automatically set by receiving an operation signal from a management medium (for example, a computer, a terminal device, or the like) that manages the subjective optometry system. In this case, it is also possible to transmit an optometry schedule to a plurality of subjective optometry devices from one management medium, change the settings of the plurality of subjective optometry devices collectively, and unify the optometry schedule.

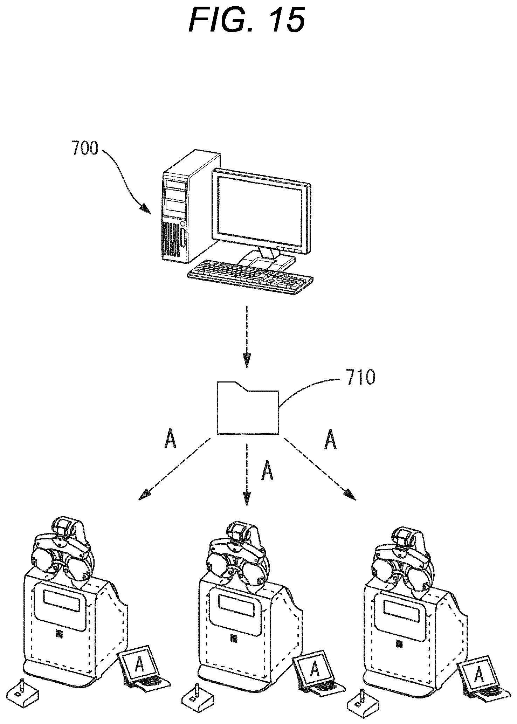

FIG. 15 is a diagram in which a plurality of subjective optometry devices are connected to a network via a terminal device. For example, a terminal device 700 may be a tablet PC, a notebook PC, a smartphone, a desktop PC, a main subjective optometry device, or the like. For example, the terminal device 700 is communicably connected to a plurality of subjective optometry devices. For example, the terminal device 700 is network-connected to the plurality of subjective optometry devices by at least one of wireless communication and wired communication. For example, the examiner creates an optometry schedule in the terminal device 700 and stores information (CSV file or the like) on the optometry schedule in a shared folder 710 on the network. For example, the control unit of each subjective optometry device acquires the information on the optometry schedule stored in the shared folder 710 on the network at the time of activation, and associates one of the first self-optometry program and the second self-optometry program with the date or the like based on the information. Accordingly, the optometry schedule can be automatically set. Therefore, the labor of the examiner is reduced. Accordingly, the optometry schedule is unified in the plurality of subjective optometry devices.

Identification information for associating at least one of the plurality of subjective optometry devices may be set in information of the optometry schedule stored in the terminal device 700. For example, identification information (as an example, an identification symbol, an identification number, or the like) unique to each subjective optometry device may be stored in association with the information of the optometry schedule (CSV file or the like). In this case, the control unit of each subjective optometry device can set a different optometry schedule for each subjective optometry device by accessing the shared folder 710 and reading the optometry schedule matching the unique identification number.

In the above description, the terminal device 700 and the network connection are used to automatically set the optometry schedule in the subjective optometry device, but the optometry schedule may be set using an external storage medium. For example, the information of the optometry schedule may be stored in a USB memory, an SD card, or the like, and may be read by each subjective optometry device. In the above description, the optometry schedule in which the first self-optometry program and the second self-optometry program are associated is set from the terminal device 700, and the examination condition of at least one of the first self-optometry program and the second self-optometry program may be set. That is, an examination item or an examination visual target of each self-optometry program may be changed and reflected in each device.

In the present example, the configuration in which the setting is automatically switched based on the optometry schedule in which the first self-optometry program or the second self-optometry program is associated with the predetermined period has been described as an example, but the present invention is not limited thereto. For example, even when the first self-optometry program is set based on the optometry schedule, the second self-optometry program may be temporarily set, and the examination items included in the second self-optometry program may be sequentially proceeded. In addition, for example, even when the second self-optometry program is set based on the optometry schedule, the first self-optometry program may be temporarily set, and the examination items included in the first self-optometry program may be sequentially advanced.

For example, the examiner can temporarily change the self-optometry program by pressing the self-optometry program switching button 224 on the self-optometry assistance screen 200 (see FIG. 7). For example, the examiner may set the second self-optometry program by pressing the self-optometry program switching button 224 in a case where the first self-optometry program is set according to the optometry schedule but it is determined that the next examinee should be examined using the second self-optometry program. The control unit 60 switches the setting to the second self-optometry program different from the current first self-optometry program, based on the input signal from the optometry program switching button 224. When the examination based on the temporarily set second self-optometry program ends and the examination result is cleared, the control unit 60 sets the first self-optometry program again. Accordingly, even when the examinee is not suitable for the current self-optometry program, the self-optometry program can be easily changed before the examination of the examinee, and the examination can be smoothly performed.

### REFERENCE SIGNS LIST

1 housing
2 presentation window
3 speaker
10 examiner controller
20 examinee controller
30 light projection optical system
40 eye refractive power measurement unit
43 examination window
60 control unit
100 subjective optometry device

## Claims

1. A subjective optometry system for subjectively measuring optical characteristics of a subject eye, the subjective optometry system comprising:
a target presentation unit configured to present an examination visual target to the subject eye;
a correction unit configured to change an optical characteristic of a target light flux emitted from the target presentation unit;
a self-optometry program setting unit configured to set either a first self-optometry program that automatically proceeds with a plurality of examination items for the subject eye based on a response input by an examinee, or a second self-optometry program that automatically proceeds with a plurality of examination items for the subject eye based on a response input by the examinee, the second self-optometry program having a different examination condition from the first self-optometry program; and
a control unit configured to control, based on the first self-optometry program or the second self-optometry program set by the self-optometry program setting unit, at least one of the target presentation unit and the correction unit to advance an optometry according to a procedure of the first self-optometry program or the second self-optometry program.

2. The subjective optometry system according to claim 1,
wherein the first self-optometry program and the second self-optometry program differ in at least one of the plurality of examination items, as the examination condition.

3. The subjective optometry system according to claim 2,
wherein the first self-optometry program includes, as the plurality of examination items, an examination item for examining a refractive power of the subject eye, and the second self-optometry program includes, as the plurality of examination items, an examination item for examining a binocular vision function of the subject eye in addition to the examination item for examining the refractive power of the subject eye.

4. The subjective optometry system according to any one of claims 1 to 3,
wherein the first self-optometry program and the second self-optometry program have different examination visual targets used in the examination item, as the examination condition.

5. The subjective optometry system according to any one of claims 1 to 4, comprising:
a setting change unit configured to change at least one examination condition of an examination condition of the first self-optometry program and an examination condition of the second self-optometry program.

6. The subjective optometry system according to any one of claims 1 to 5, comprising:
an optometry schedule setting unit configured to set an optometry schedule in which the first self-optometry program and the second self-optometry program are each associated with a predetermined period,
wherein the self-optometry program setting unit sets to switch between the first self-optometry program and the second self-optometry program, based on the optometry schedule set by the optometry schedule setting unit.

7. The subjective optometry system according to claim 6, comprising:
an operation unit configured to input an operation signal for setting at least one of the first self-optometry program and the second self-optometry program,
wherein the optometry schedule setting unit sets the optometry schedule in which the first self-optometry program and the second self-optometry program are each associated with a predetermined period, based on the operation signal from the operation unit.

8. A subjective optometry program used in a subjective optometry system for subjectively measuring optical characteristics of a subject eye, the subjective optometry system including
a target presentation unit configured to present an examination visual target to the subject eye, and
a correction unit configured to change an optical characteristic of a target light flux emitted from the target presentation unit,
the subjective optometry program comprising an instruction of performing:
a self-optometry program setting step of setting either a first self-optometry program that automatically proceeds with a plurality of examination items for the subject eye based on a response input by an examinee, or a second self-optometry program that automatically proceeds with a plurality of examination items for the subject eye based on a response input by the examinee, the second self-optometry program having a different examination condition from the first self-optometry program; and
a control step of controlling, based on the first self-optometry program or the second self-optometry program set in the self-optometry program setting step, at least one of the target presentation unit and the correction unit to advance an optometry according to a procedure of the first self-optometry program or the second self-optometry program.
